# EUROPEAN PATENT APPLICATION

(11) **EP 3 998 275 A1**
(43) Date of publication of application: **18.05.2022**
(21) Application number: 20836980.1
(22) Date of filing: 10.07.2020
(51) Int. Cl.: C07K 7/08, A61K 38/10, G01N 33/531, G01N 33/574, A61P 35/00

(54) **RECOMBINANT PEPTIDES THAT BIND TO A TUMOUR-SPECIFIC ANTIBODY FOR BREAST CANCER AND USE**

(30) Priority: 10.07.2019 BR 102019014302
(71) Applicant: Universidade Federal de Uberlândia, 38400-902 Uberlândia - MG (BR); Fundação de Amparo à Pesquisa do Estado de Minas Gerais - Fapemig, 31035-536 Belo Horizonte - MG (BR)
(72) Inventor: ARAÚJO, Thaise Goncalves De, 38702-176 Patos de Minas, MG (BR); MAIA, Yara Cristina de Paiva, 38412-641 Uberlandia, MG (BR); ALVES, Douglas Alexsander, 38408-200 Uberlandia, MG (BR); MOTA, Sara Teixeira Soares, 38405-329 Uberlandia, MG (BR); ARAUJO, Galber Rodrigues, Viena (AT); VAZ, Emilia Rezende, 38411-449 Uberlandia, MG (BR); RIBIERO, Matheus Alves, 38703-194 Patos de Minas, MG (BR); ZOIA, Mariana Alves Pereira, 38408-056 Uberlandia, MG (BR); VECCHI, Lara, 38406-064 Uberlandia, MG (BR); FILHO, Luiz Ricardo Goulart, deceased (BR)
(74) Representative: IP Sextant s.r.l.
(86) International application number: PCT/BR2020/050252
(87) International publication number: WO 2021/003545

(57) **Abstract**

The present invention refers to the use of synthetic peptides built from the selection of peptides available on the surface of filamentous phages and that bind to the FabC4 antibody fragment with clinical relevance in the diagnosis of Breast Cancer (BC) and in the prognosis of Triple-Negative Breast Cancer (TNBC). The peptides were specific to tumor proteins and could be potentially used in immunodiagnostic tests, formulations coupled to fluorescent or radioactive particles for diagnostic imaging, therapeutic drug carriers, and in immunogenic compositions for BC management. The technology used for peptide selection was the peptide expression system on the surface of filamentous bacteriophage. The strategy used was the selection of binding peptides of tumor proteins present in serum from TNBC patients.

Sequence Listing

<110>  Universidade Federal de Uberlândia
       FAPEMIG

<120>  BREAST CANCER SPECIFIC TUMOR ANTIBODY BINDING RECOMBINANT PEPTIDES
AND RELATED USE

<130>  Diagnosis and treatment of triple-negative breast cancer

<160>  4

<170>  PatentIn version 3.5

<210>  1
<211>  12
<212>  PRT
<213>  Homo sapiens

<400>  1

Leu Thr Pro Leu Thr Ser Pro Gly Thr Leu Leu Arg
1               5                   10

<210>  2
<211>  12
<212>  PRT
<213>  Homo sapiens

<400>  2

Arg Tyr Leu Pro Thr Phe Asp Met Val Ser Arg Thr
1               5                   10

<210>  3
<211>  12
<212>  PRT
<213>  Homo sapiens

<400>  3

Val Met Pro Ala Ala Lys Tyr Ser Leu Arg Val Leu
1               5                   10

<210>  4
<211>  12
<212>  PRT
<213>  Homo sapiens

<400>  4

Val Thr Pro Cys Ser Ser Phe Ser Ser Phe Leu Pro
1               5                   10

## Description

### Field of invention

. The present invention refers to the use of synthetic peptides built from the selection of peptides available on the surface of filamentous phages and that bind to the FabC4 antibody fragment with clinical relevance in the diagnosis of Breast Cancer (BC) and in the prognosis of Triple-Negative Breast Cancer (TNBC) (Araújo T.G. et al., Cancer Lett., 2014.). The peptides were specific to tumor proteins and could potentially be used in immunodiagnostic tests, formulations coupled to fluorescent or radioactive particles for diagnostic imaging, therapeutic drug carriers, and in immunogenic compositions for BC management. The technology used for the selection of peptides was the peptide expression system on the surface of filamentous bacteriophage. The strategy used was the selection of binding peptides of tumor proteins present in serum from TNBC patients.

### State of the art

. Breast Cancer (BC) is considered the most common type of tumor in women, with about 1.7 million new cases diagnosed in 2012. It represents 25% of all cancers that affect women and is the fifth largest worldwide cause of death from the disease (WCRF- World Cancer Research Fund International, 2018). In Brazil, excluding non-melanoma skin cancer, breast cancer is the most frequent type of cancer in women, accounting for 28% of new lesions identified annually. Around 59,700 new cases are expected for the 2018-2019 biennium. It is estimated that in Brazil there were 15,403 deaths from this neoplasm in 2015 (INCA, 2018). It is estimated that in Brazil 15,403 deaths from this neoplasia occurred in 2015 (INCA, 2018).

. Breast Cancer is a predominantly genetic disease, in which genomic changes inherited or resulting from mutations throughout life lead to malignant transformation. Several factors have been correlated with the onset of this disease, such as: genetic predisposition related to the presence of mutations in the BRCA1 (Breast Cancer 1) and BRCA2 (Breast Cancer 2) genes; the age; endocrine characteristics/reproductive history of the woman, including hormonal stimulation, early menarche, late menopause and nulliparity; and environmental/behavioral factors such as alcohol consumption, overweight and exposure to ionizing radiation (INCA, 2018; Anderson K. N. et al., Breast Cancer Research and Treatment, 2014).

. After diagnosis, they are classified and staged. Since its creation in 1977, the staging standard proposed by the International Union Against Cancer (IUAC) is still widely used. The TNM system is based on anatomical findings and analyzes three main aspects: tumor size (T), lymph node involvement (N) and the occurrence or not of metastases (M) (Giuliano A. E. et al., Ann. Surg. Oncol., vol. 25, no. 7, pp. 1783-1785, 2018).

. However, they are highly heterogeneous tumors from a clinical, biological, and morphological point of view. Three main molecular subtypes are described, with different prognostic profiles and therapeutic strategies. Microarray gene expression assays and immunohistochemistry experiments subclassify mammary tumors into: luminal (A and B); those that overexpress the human epidermal growth factor receptor 2 (HER-2) and those that are triple-negative. In addition, the "claudin-low" and the apocrine molecular are also described (De Barros A.C.S. and Leite K. R. M., Rev. Bras. Mastol, 2015).

. The triple-negative tumor lacks estrogen receptor (ER), progesterone receptor (PgR) and HER-2. They are generally aggressive, have a worse prognosis and account for about 15% of diagnoses (Pala E. E. et al., Pathol. Oncol, 2015). Furthermore, they are morphologically, genetically, immunophenotypically and clinically divergent, in addition to not having a specific therapeutic strategy already defined (Carey L. A. et al., Clin. Cancer Res., 2007). There are different protein and mRNA expression profiles, making it difficult to define a single triple-negative BC (TNBC) (Gluz O. et al., Ann. Oncol., 2009). Therefore, the difficulty of systematically classifying triple-negative tumors is evident.

. In such a context, numerous efforts are made in the search for new and promising therapeutic approaches, as well as strategies that can better elucidate this tumor subtype (Lehmann B. D. B. et al., J. Clin. Invest., 2011.).

. One of the great challenges in BC is the development of specific therapies for the triple-negative subtype (Collignon J. et al., Breast cancer, Dove Med. Press, 2016). Tomao *et al.* (2015) described the existence of molecular characteristics inherent to such tumor that can be therapeutically approached. Phage Display, first described in 1985, searches for peptides binding to specific targets using viral vectors (Scott J. K. and Smith G. P., Science, 1990). This technique proves to be promising in mapping key epitopes to unravel the mechanisms inherent in the emergence and progression of various diseases, as it does not require prior knowledge of their targets (Moreira G. M. S. G. et al., in Methods in molecular biology, 2018).

. Our research group characterized in a previous study, through Phage Display, a new FabC4 antibody with clinical relevance in the diagnosis of BC and in the prognosis of TNBC (T. G. Araújo et al., Cancer Lett., 2014). Recombinant antibody was associated with younger age, absence of the progesterone receptor, higher histological grades, and non-luminal phenotype. In addition, it identified a subgroup of triple-negative BC with a good prognosis. Therefore, further characterization of its target is essential for the clinical management of said neoplasia.

. Given the heterogeneous, multifactorial, and multifocal nature of BC, the search for potential biomarkers and their molecular associations involved in the occurrence and development of this disease is essential for a more accurate diagnosis and to clarify the neoplastic phenotype (Mittal S. etal., Biosens. Bioelectron., 2017). Recently, several studies have been carried out aimed at the identification and validation of antigens for tumor detection, especially in the search for serum biomarkers (Gerasimov E. et al., BMC Bioinformatics 2017).

. We then performed a bioprospecting assay against FabC4 using a PH.D-12 library in order to map its epitopes. We selected and characterized phage clones that mimic epitopes (mimotopes) corresponding to the proteins Annexin A2 and Cytokeratin 10 (CK10). Furthermore, our results have opened new perspectives in BC diagnosis and in the development of new therapies.

. Considering the diagnosis, patent WO2013075059A1 refers to the development of a diagnostic method of TNBC based on gene expression profile, which are biomarkers for this tumor subtype. The disadvantage of this model, compared to the one presented in this application, is the high cost and the need to evaluate an extensive set of genes.

. Phage Display technology has great relevance in the development of biomolecules with diagnostic and therapeutic potential. First described in 1985, it consists of the search for binding peptides to specific targets through the use of viral vectors (Scott J. K. and Smith G. P., Science, 1990). This technique proves to be promising in mapping key epitopes to unravel the mechanisms inherent in the emergence and progression of various diseases, as it does not require prior knowledge of their targets (Moreira G. M. S. G. et al., in Methods in molecular biology, 2018).

. Patents WO2002020723A2, WO2002020822A2, WO2002020722A2 refer to the isolation of peptides binding the surface of tumor cells, by Phage Display, exposing their use in *in vitro* and/or *in vivo* compositions in humans.

. In the search for a fast, practical, efficient methodology capable of strengthening the diagnostic methods currently used for breast cancer, for targeting the triple-negative tumor subtype, and providing a platform for a more effective treatment for TNBC, the present invention proposes protection for the use of peptide sequences (Seq. ID No. 1 to Seq. ID No. 4), whether fused or not to carriers, such as fluorescent/radioactive compounds, drugs, filamentous phages, or any other types of carriers, whether associated with radioisotope labeling, conjugated to fluorophores in general, Alexa-fluor variants with their different wavelengths, the GFP (green fluorescent protein), PEG (polyethylene glycol), biotin, quantum-dots, or conjugated to chemical groups, nanomaterials and chemotherapeutic molecules or used together with labeled secondary antibodies in general for detection, in order to increase stability and use in diagnostics and/or treatment of breast cancer in general and in the triple-negative subtype, as well as in other applicable diseases. We claim the use of these peptides in enzyme immunoassays, such as ELISA, immunohistochemistry, immunoagglutination and immunophenotyping tests, electrochemical sensors, HPLC flow cytometry, or any other form of detection directly or indirectly related to blood, urine or saliva samples.

. It is also intended to protect the use of peptides conjugated to fluorescent, colorometric, radioisotopes, luminescent agents, nanocrystals, magnetic nanoparticles, and others for obtaining *in vitro* or *in vivo* images, detection by fluorescence, luminescence, or colorimetry in static or portable equipment, and visualization under a microscope, as well as formulating immunogenic compositions containing one or more compounds defined by the peptide sequences (Seq. ID No. 1 to Seq. ID No. 4), and any physiologically and pharmaceutically acceptable adjuvants.

. The invention will be better explained by means of the detailed description through examples 1, 2, 3, 4, 5, 6 and 7 of the experimental results obtained. Experimental procedures are detailed in the topic "Experimental Methodology" at the end of this descriptive report. The approach performed in the selection, characterization and use of peptides was applied to samples from patients with BC, Benign Breast Disease (BBD) and Normal Control (NC), proving the applicability of its use in the diagnosis and possible treatment of TNBC.

### Example 1

. This example concerns the selection of peptides binding to the FabC4 antibody fragment and mimetics of their targets. In order to achieve this objective, a bioprospecting assay was carried out using Phage Display technology with the use of bacteriophage viruses that expressed, on the surface of their capsid, random peptides of 12 amino acids for the selection of FabC4-specific clones. After selection, Phage-ELISA assays were performed to verify which bacteriophages were more specific for FabC4.

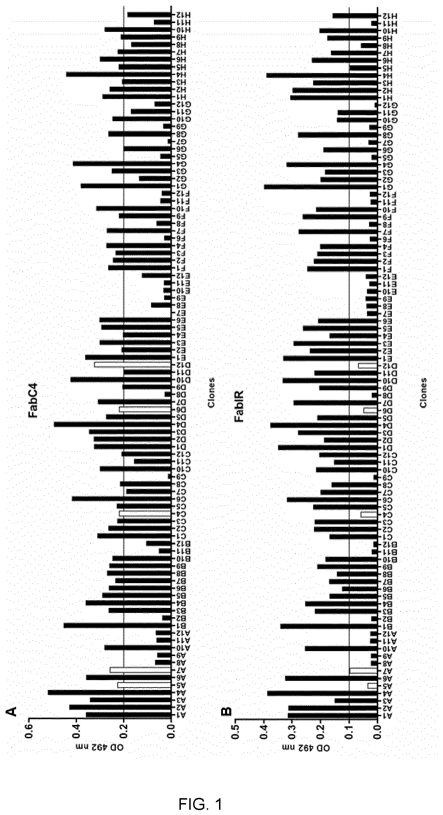

. FIGURE 1 shows the representative plot of the different reactivity of the clones to FabC4 and FabIR (irrelevant, used as a negative control). Although several clones showed high reactivity to FabC4 (absorption above wild-type phage), only five were selected, considering the parameter based on absorbance above 0.2 for FabC4 and below 0.1 for FabIR. This strategy allowed the identification of phages with high affinity for FabC4.

### Example 2

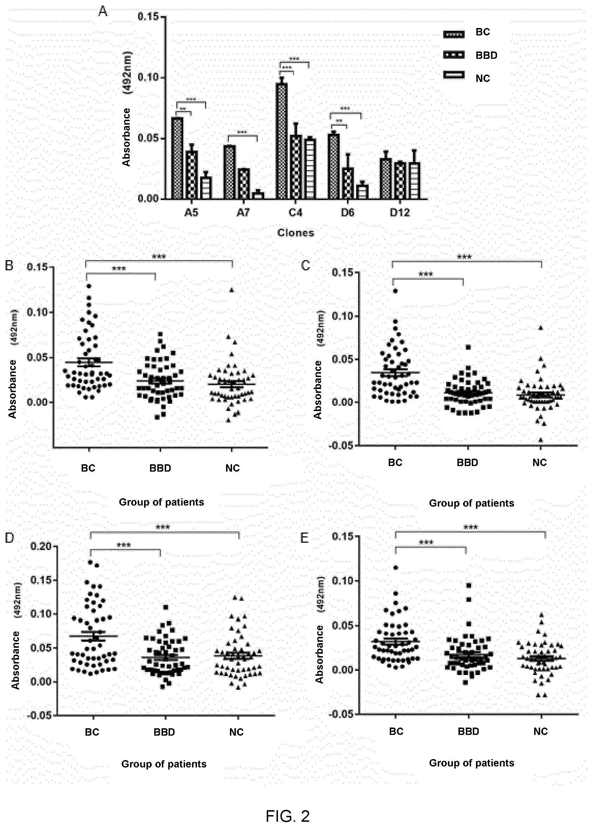

. In order to investigate the sensitivity of these phages to circulating IgGs from patients with BC, BBD and NC and their possible roles as biomarkers, enzyme immunoassays were carried out in two stages, concentrated in FIGURE 2. First, three pools of sera consisting of 10 samples from each group were used for a preliminary evaluation of the five chosen clones, shown in Figure 2A. Phagotope reactivity in pooled sera significantly differentiated BC from BBD group in three of the five clones tested. Phage A7 was more reactive to BC serum only when compared to NC patients (P<0.001).

. In a second moment, phages A5, A7, C4 and D6 were conducted in ELISA assays with a larger number of patients (50 from each group). All values were normalized to the absorbance obtained for wild-type phage. The results demonstrated a successful affinity selection of four mimotopes capable of significantly recognizing IgGs present in the serum of patients with BC.

. All selected clones were able to discriminate tumor samples from benign breast disease and normal samples. The relative absorbances of phages A5, A7, C4 and D6 are shown in FIGURES 2A-2B-2C-2D-2E respectively. For none of the clones there were statistically significant differences between BBD and NC samples.

. ROC curve was then constructed to predict the overall value of each phage selected as a diagnostic tool. The area under the curve (AUC) was calculated, as well as the sensitivity, specificity, and likelihood ratio, shown in TABLE 1. As in the ELISA assay, the four clones showed similar behavior with AUC above 0.70, except the C4 phage when their data for BBD were compared to NC.

**TABLE 1**

| | **Phage AS** | | **Phage A7** | | **Phage C4** | | **Phage C4** | |
|---|---|---|---|---|---|---|---|---|
| | BC × BBD | BC × NC | BC × BBD | BC × NC | BC × BBD | BC × NC | BC × BBD | BC × NC |
| **AUC** | 0.702 | 0.756 | 0.767 | 0.805 | 0.706 | 0.683 | 0.71 | 0.74 |
| **p-valor** | 0.00052 | <0.0001 | <0.0001 | <0.0001 | 0.0004 | 0.0016 | 0.00030 | <0.0001 |
| **Cut-off** | <0.043 | <0.043 | <0.028 | <0.028 | <0.078 | <0.078 | <0.0295 | <0.0295 |
| **Sensibility** | 82% | 92% | 88% | 92% | 94% | 86% | 80% | 86% |
| **Specificity** | 38% | 38% | 50% | 50% | 40% | 40% | 42% | 42% |
| **Likelihood** | 1.32 | 1.48 | 1.76 | 1.84 | 1.56 | 1.43 | 1.38 | 1.48 |

| | | | | | | | | |
|---|---|---|---|---|---|---|---|---|
| AUC: Area Under the Curve; BC: Breast Cancer; BBD: Benign breast disease; NC: Normal Control (healthy women) | | | | | | | | |

. Clone A7 stood out for the set of values: AUC=0.767 (P=0.01), sensitivity of 88% and specificity of 50%, comparing BC with BBD and AUC=0.805 (P=0.01), sensitivity of 92% and specificity of 50%, comparing BC and NC. Clone A5 showed a lower specificity when differentiating malignant patients from those diagnosed with benign disease. Odds ratio (OR) analysis supports the predictive value of our A5 phages (OR = 4.32 (95% Cl 1.8 to 9.8), P = 0.0004), A7 (OR = 7.78 ( 95% Cl 3.36 to 17.23), P = 0.0001), C4 (OR = 6.52 (95% Cl 2.78 to 14.95), P = 0.0001) and D6 (OR = 4.66 (95% Cl 2.22 to 9.59), P = 0.0001), which attribute a greater chance of the patient having a tumor when discriminated in the enzyme immunoassay.

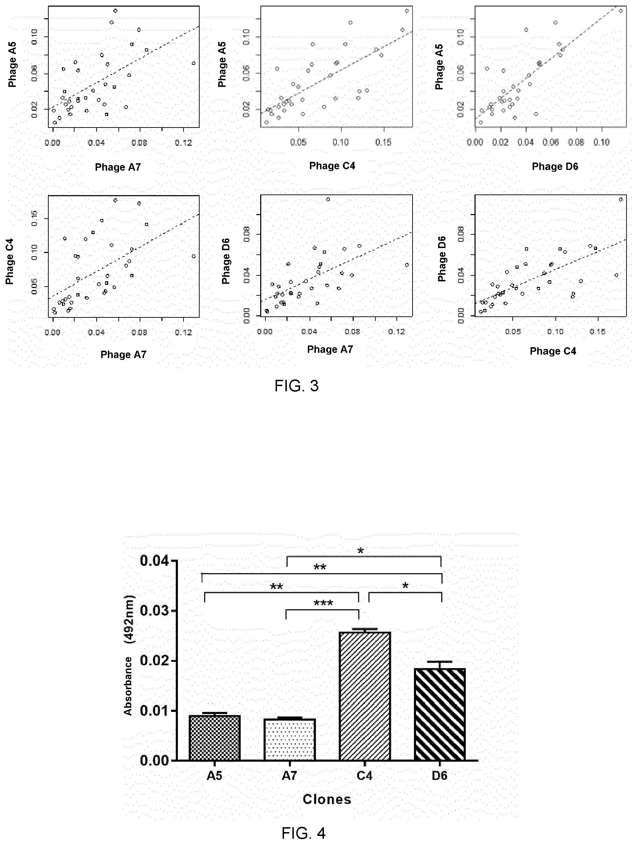

. The four selected phages exhibited a similar reactivity profile in ELISA results. The correlation between the absorbances for the BC group was then evaluated, in order to demonstrate the synergism in the behavior of these clones. As seen in FIGURE 3 the linear correlation analyzes corroborated this hypothesis. We found a significant positive correlation in all analyzes (p<0.05). For each of the clone pairs: A5xA7 (R= 0.5917, p= 0.000228), A5xC4 (R= 0.734, p= 7.7e-07), A5xD6 (R= 0.806, p= 8.841e-09), C4xA7 (R= 0.554, p= 0.000127), D6xA7 (R= 0.61, p= 0.000127) and D6xC4 (R= 0.71, p= 2.62e-06).

### Example 3

. Circular ssDNA fragments from the four validated clones were extracted, sequenced, and translated using the ExPASy program. Different sequences were obtained, shown in TABLE 2. The analyzes in the SAROTUP database confirmed the non-redundancy of said sequences.

**TABLE 2**

| Seq. ID | Clone | Sequence |
|---|---|---|
| Seq. ID No. 1 | A5 | LTPLTSPGTLLR |
| Seq. ID No. 2 | A7 | RYLPTFDMVSRT |
| Seq. ID No. 3 | C4 | VMPAAKYSLRVL |
| Seq. ID No. 4 | D6 | VTPCSSFSSFLP |

. Once the objective was to map the FabC4 epitope, the peptides were then chemically synthesized (GenScript) and evaluated for their ability to bind to the FabC4 antibody fragment by ELISA, seen in FIGURE 4. All were reactive to the target. Peptide pC4 showed the highest absorbance when compared to the others (p<0.05 for all comparisons), followed by pD6 (also p<0.05 for all comparisons).

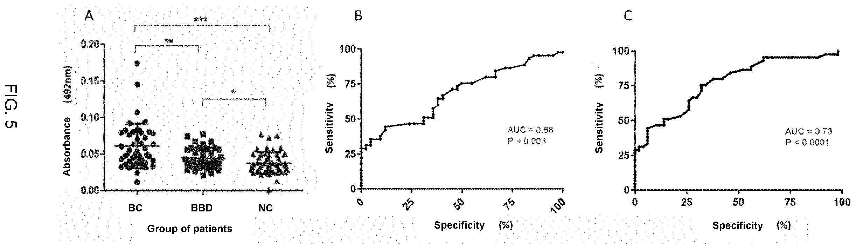

. The four peptides were then tested for their diagnostic ability against serum from 150 patients (50 from each study group). Exemplified by the synthetic peptide pD6, it is observed that it showed differential reactivity between the samples, as shown in FIGURE 5A. FIGURES 5B and 5C show that the ROC curve was significant for BC × BBD (AUC= 0.68) and BC × NC (AUC = 0.78), with sensitivities of 88.10% and 44.4%, and specificities of 94% and 44.4%, respectively, confirming the potential for diagnostics.

### Example 4

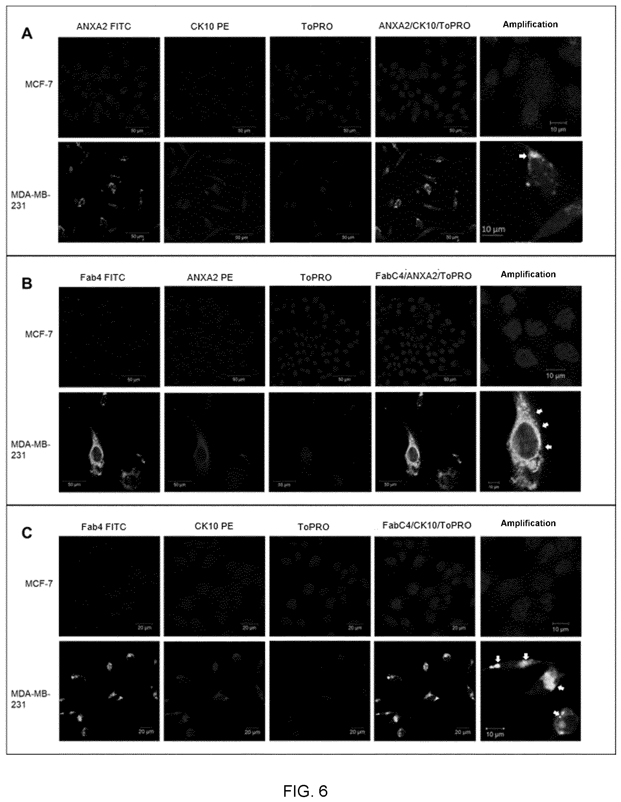

. This example refers to the validation of the ability of FabC4 antibody to bind to ANXA2 and CK10 targets, shown in FIGURE 6. Initially, the behavior of these proteins in the MCF-7 (luminal phenotype) and MDA-MB-231 (triple-negative phenotype) tumor cell lines was evaluated, FIGURE 6A-B. ANXA2 was found predominantly in the cytoplasm of MCF7 cells. This staining was less intense than in MDA-MB-231, seen in FIGURE 6A-B.

. Identified in the cytoplasm, the staining for CK10 was also different between the two strains, being more intense in MDA-MB-231. In these cells, the protein was also visualized in the nucleus. Interestingly, in this cellular model, the two proteins colocalized in the cytoplasm, visualized in FIGURE 6A-arrow, a behavior that has not yet been described in mammary tumors.

. Marking for FabC4 was cytoplasmatic and more intense in the MDA-MB-231 lineage, following the profile of its targets, FIGURE 6B-C. The colocalization with ANXA2, shown in FIGURE 6B-arrow and CK10 in FIGURE 6C-arrow, confirm the ability of this recombinant antibody to recognize these proteins.

### Example 5

. This example refers to three-dimensional modeling, interaction (Docking) between the selected peptides and FabC4, and three-dimensional alignment to characterize, *in silico,* FabC4 epitopes. To better elucidate the interactions and as a function of the correlations observed in the Phage-ELISA assays, peptides (pA5, pA7, pC4 and pD6) were modeled individually and in combinations.

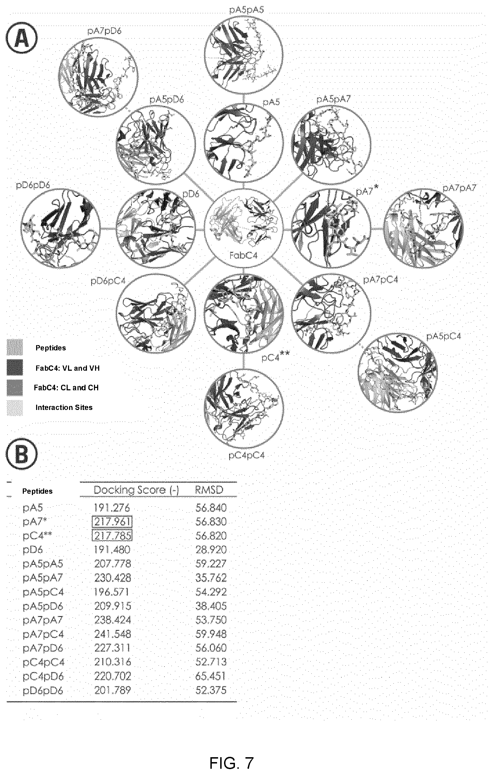

. Peptides were modeled by PEPFOLD 2.0 software, while FabC4 was modeled by RAPTORX. PEPFOLD 2.0 provided 100 most likely models for each of the four peptides and their combinations. The best individual result was submitted as input in .pdb format, along with the modeled three-dimensional structure of FabC4, in HPEPDOCK online tool. This software generated another 100 simulation models of FabC4-Peptide interactions and their combinations, which were individually ranked and evaluated by the Fast Interaction REfinement in molecular DOCKing (FIREDOCK) software. The best is shown in FIGURE 7A.

. RMSD (Root Mean Square Deviation of atomic positions) values were used to determine how how specific and consistent are the predictions and the interaction positions at the FabC4 site. In FIGURE 7B, we highlight the peptides that presented the best score, evaluated in terms of coherence and physical-spatial stability. In this way, pA7 and pC4 peptides were more likely to interact with FabC and therefore selected for subsequent analyses.

### Example 6

. This example refers to the ability of peptides to mimic ANXA2 and CK10 proteins. Considering ANXA2 (PDB:1W7B) and CK10 (PDB:4ZRY) as FabC4 targets, it was necessary to validate, *in silico,* the ability of the selected peptides to mimic them.

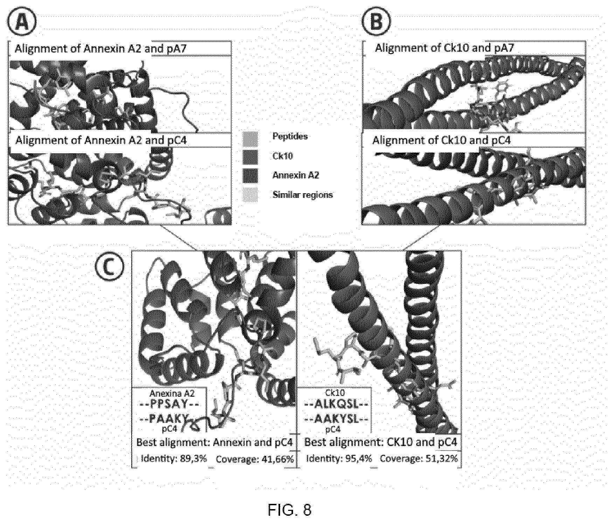

. The alignment by overlapping and comparison of amino acids were performed in the PyMOL program, locating the regions with the highest identity (> 30%) and coverage. After analysis, results showed that pC4 (Peptide expressed in phage C4) mimicked ANXA2 and CK10 in two different positions, as seen in Figure 8. In FIGURE 8A the alignment between the peptides and ANXA2 is noted, while in FIGURE 8B it is possible to see the three-dimensional identity between peptides and CK10. The best alignments for pC4, as well as the description of similar amino acids between the structures are shown in FIGURE 8C. For the first, the mimicked region was PPASY (Proline-Proline-Alanine-Serine-Tyrosine) and for the second it was ALKQSL (Alanine-Leucine-Lysine-Glutamine-Serine-Leucine).

### Example 7

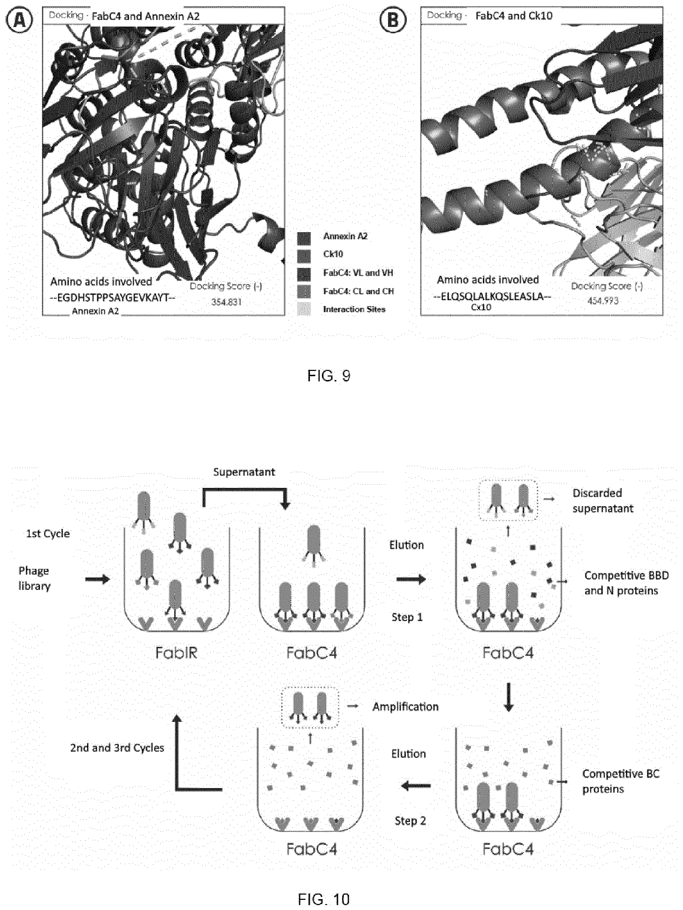

. This example refers to the proof of success of peptides as mimetics of FabC4 epitopes. For this, ANXA2 and CK10 targets were modeled and anchored (molecular docking) to the FabC4 antibody, and the interaction regions were compared to those of the pC4-FabC4 complex. Protein dockings were performed using the PATCHDOCK 1.3 software, without targeting site, so that the simulations covered the entire surface of the binder. The software provided ten models, which, also by FIREDOCK, were thermodynamically classified by the best score. FIGURE 9A shows the representation of the interactions of FabC4 with ANXA2, and in FIGURE 9B with CK10. The yellow dashed part indicates the non-covalent interaction between amino acids. The structures proved to be stable and through FIGURE 9 it is possible to observe that the amino acids involved in the FabC4-ANXA2 and FabC4-CK10 interactions coincided with the amino acids involved in the FabC4-pC4 interaction, confirming the successful selection of mimetic peptides to the epitope of the antibody fragment of interest.

### The used methodology

. For a better understanding of the examples used in the present invention, this topic follows with a detailed description of the procedures adopted.

### Total protein extraction from patients

. Patient tissue fragments were macroscopically dissected and macerated in liquid nitrogen using a crucible with a porcelain stick in extraction buffer (20mM Tris-HCL pH7.2, 10mM EDTA, 2mM EGTA, 250mM sucrose, 1mM DTT, 1mM Benzamidine). The resulting material was transferred to a microtube and centrifuged at 20,000 × g for 30 min at room temperature. Supernatant was collected and the total protein concentration was quantified by Bradford method ^{[23]}. To preserve its integrity, benzamidine protease inhibitors (100 mM) and phenylmethylsulfonyl fluoride (PMSF) (150 mM) were added. Proteins were extracted from the tissues of patients (five for each group) diagnosed with BC, Benign Breast Disease (BBD) and samples from mammoplasty, considered normal (NC).

### Phage display

. A library of random 12 amino acid peptides fused to the minor coat protein (pill) of bacteriophages M13 - PhD-12 (New England Biolabs, Beverly, MA, USA) was used. In this bioprospection approach, 1,5 × 10¹¹ viral particles were used in three selection cycles against FabC4 antibody, amplified as previously described ^{[24]}, with modifications. The experimental design is shown in FIGURE 10.

. For selection, 10µg of FabC4 and FabIR were used, immobilized in a 96-well plate for 24h at 4°C. Blocking was performed for 1h at 37°C with PBS1X-BSA3%, followed by two washes with 300µL of TBS-T0.1% (Tris-Buffered Saline and 0.1% of Tween 20). In a subtractive selection strategy, FabIR was incubated with 1,5 × 10¹¹ phage particles genetically modified, for peptide expression on their surface, from PhD-12 library, in 200µL of TBS solution at room temperature (RT) for 30 min. Subsequently, supernatant containing the non-binding phages were exposed to FabC4 for 1 hour at RT.

. After this period, the well containing FabC4 was washed ten times with TBS-T0.1% and the particles that did not bind were discarded. Then the recombinant phages that recognized FabC4 were disconnected from the target by means of a two-step elution aiming to select BC-specific and related peptides. In a first moment, the FabC4-Phago complex was incubated with proteins extracted from benign and normal tissues (30 min at RT) competitively eluting the mimetic peptides to non-tumor proteins. These were discarded. Viruses that remained bound to FabC4 were subjected to a second competitive elution with proteins extracted from tissues with BC (30 min at RT), thereby unbinding the peptide mimetics to tumor proteins. These were amplified in Escherichia coli ER2738 (New England Biolabs, Beverly, MA, EUA), purified with PEG-NaCl (20% of Polyethylene Glycol 8000 and 2.5M of NaCl - sterile solution) and used in a new selection cycle. From the second round, the stringency of the wash buffer was increased from 0.1% to 0.5% of Tween 20. After the third round, individual bacterial colonies containing amplified phage clones were grown on a deep well plate for validation of biopanning and the selection of promising clones.

### Amplification and purification of recombinant phages

. Phages were amplified using a colony of E. coli from lineage ER2738 previously inoculated in 20mL of sterile Luria-Bertani (LB) medium containing tetracycline (20 µg/mL); kept under stirring at 37°C until OD600nm = 0.3. This culture was diluted in new LB medium containing tetracycline (1mL of culture/120mL of medium) and it was distributed in a deep well plate (1mL/well).

. Using sterilized sticks, blue colonies (phage-infected bacteria) were removed from the petri dish (referring to the non-amplified third round) and transferred to a well of the deep well plate containing the previously diluted culture transferred to a well of the deep well plate containing the previously diluted culture, which was sealed with an adhesive and incubated for 5 hours under strong agitation at 37°C. For safety, 100µL of each well was stored in a microtiter plate (Falcon^{®} -Thermo Fisher), and 100µL of 50% of glycerol was added. After incubation, the deep well plate was centrifuged for 20 minutes at 3700 rpm and supernatants were transferred to another sterile plate, to which 1/6 of the volume in PEG/NaCl was added and incubated for 14 hours at 4°C. After this period, the plate was centrifuged for 1 hour at 3700 rpm and all the supernatant was discarded. The precipitate was resuspended in 200µL of PBS1X.

### DNA extraction and sequencing

. For phage DNA extraction, 10µl of bacteria infected with each clone were inoculated into 1mL of LB medium in Deep well plates, which were incubated for 16 hours at 37°C under stirring and then centrifuged at 3700 rpm for 50 min at 4°C. Supernatant was then transferred to a new plate and 1/6 of the volume of PEG/NaCl was incorporated and kept at 4°C for 10 min. After further centrifugation for 1 hour at 3700 rpm at 4°C, the supernatant was discarded and 100µl of iodide buffer (10mM of Tris-HCI pH 8.0, 1mM of EDTA and 4M of Nal) were added to each well. After brief stirring, 250µl of absolute ethanol were added and the solution was incubated for 10 min at RT. Subsequently, the plate was centrifuged for 40 min at 3700 rpm at 20°C. The supernatant was discarded, 150µl of 70% ethanol was added and centrifuged again for 10 min at 3700 rpm. The supernatant was discarded, and the phage DNA was resuspended in 15ul of ultrapure water.

. Sequencing reactions were carried out in ABI3500 automatic sequencer (Applied Biosystem, California, USA), following the recommendations of the manufacturer and using the primer -96M13 (5'-CCCTCATTAGTTAGCGCGTAACG-3'). Amino acid sequences were deduced from the nucleotide sequences using the ExPASy software (http://www.expasy.org). An analysis of the peptides was also performed using the SAROTUP software (http://immunet.cn/sarotup/index.html) to validate the peptides as non-binding to the adsorption surfaces. Alignment via BLASTp (NCBI) allowed the similarity analysis of the selected peptides.

### FabC4 reactivity

. To confirm the recognition of the selected peptides by the target molecule, Phage-ELISA assays were performed in duplicate. 96-well microtiter plates (Nunc MaxiSorp-Sigma^{®}) were coated with 1µg/well of FabIR and FabC4 diluted in carbonate/bicarbonate buffer (50mM pH 9.6); kept for 16 hours at 4°C. The wells were washed with PBS-T0.1% and blocked for 1h at 37°C with PBS-BSA3%.

. Subsequently, the plate was washed twice with PBS-T0.1% and the culture supernatant containing amplified viral particles (~1010 pfu/mL) was added, with subsequent incubation for 1h at 37°C. Four washes were performed with PBS-T0.1% and then the anti-M13 antibody that is conjugated to peroxidase diluted 1:5000 in PBS1X-BSA3% was pipetted. The plate was kept for 1h at 37°C followed by four washes with PBS-T0.1%.

. The reaction with the OPD (o-phenylenediamine dihydrochloride) substrate SigmaFast^{®} (Sigma-Aldrich), interrupted with H2SO4 (2N), was analyzed in a spectrophotometer at a length of 492nm (TP-Reader ThermoPlate). M13 phage without displaying any peptide (wild) was used as a negative control and normalizer of the obtained values. For further assays, phages whose reactivity to irrelevant was less than 0.1 and to FaC4 was greater than 0.2 were chosen.

### Phage-ELISA in patient serum

. ELISA assays were performed to identify the antigens as circulating. Polystyrene microplates (Nunc MaxiSorp-Sigma^{®}) were coated with 10¹⁰ pfu of each phage clone diluted in carbonate/bicarbonate buffer (50mM pH 9.6), incubated for 16 hours at 4°C. After sensitization, the microplates were washed once with PBS-T0.05% and blocked at 37°C for 1 hour with 300µL of PBS-T0.05%M5% (PBST0.05% plus 5% skimmed milk - PBSTM). Then, serum samples diluted 1:400 in PBSTM were incubated for 1 hour at 37°C. Three pools of serum corresponding to each of the groups were used: BC (10 patients with invasive ductal carcinoma); BBD (10 patients) and NC (10 patients).

. Plates were washed five times with PBS-T0.1% and the anti-human IgG antibody which was labeled with peroxidase (Sigma) diluted in 1:5000 in PBSTM was added. After 1 hour of incubation at 37°C and washing with PBS-T0.1%, the OPD substrate was added, and the reaction was stopped with H2SO4 (2N). Optical densities were determined at 492 nm in an ELISA reader (TP-Reader ThermoPlate) and the experiments were carried out in triplicate.

. Absorbances were normalized with the values obtained for wild-type M13 phage, used as control. Four clones were then individually tested in triplicate against a new group of 150 patients (50 BC; 50 BBD; 50 NC). The optimal reaction point (cut-off) was determined based on the ROC curve data.

### Synthetic peptides as epitopes of fabc4

. Clones A5, A7, C4 and D6, which were able to discriminate the serum of individuals with BC from BBD and NC by Phage-ELISA, were chemically synthesized by GenScript USA Inc. To increase immunogenicity, the peptides were coupled to BSA. Enzyme immunoassays were conducted to assess the ability of FabC4 antibody to recognize these peptides.

. A microtiter plate was sensitized with 2.5 µg/well of each of the peptides diluted in carbonate/bicarbonate buffer (50mM pH 9.6) and kept for 16 hours at 4°C. After blocking with PBS1X-BSA3% (1 hour at 37°C) a single wash with PBS1X preceded the addition of FabC4 (1ug), incubated for 1 hour at 37°C. After washing with PBS1X, anti-HA antibody which was labeled with peroxidase (Roche Applied Science) diluted 1:5000 in PBS-BSA3% for 1h at 37°C was added. After washing with PBS1X, the reaction with the OPD SigmaFast^{®} (Sigma-Aldrich) substrate, interrupted with H2SO4 (2N), was analyzed in a spectrophotometer at a length of 492 nm (TP-Reader ThermoPlate).

. The same conditions were used to analyze the diagnostic capacity of the peptides against the serum of the 150 patients previously described. Again, ANOVA and Mann Whitney tests were used for comparative purposes between groups with the aid of the GraphPad Prism 7.0 (GraphPad Software Inc. - La Jolla, CA, USA) software. Values of P<0.05 were considered significant.

### Molecular modeling and docking

. To determine the three-dimensional structure of FabC4, as well as the possible interactions and links between the peptides and the recombinant antibody, *in silico* molecular modeling and docking assays were performed. The three-dimensional structure of the FabC4 antibody fragment was modeled from the FASTA construction of the light and heavy chains of the variable and constant portions using RaptorX software (http://raptorx.uchicago.edu/StructurePrediction/predict/). The model was chosen based on the scores presented, as well as with the help of the Verify3D software (http://servicesn.mbi.ucla.edu/Verify3D/) and RAMPAGE: Ramachandram Plot Assessment (http://mordred.bioc.cam. ac.uk/~rapper/rampage.php).

. The peptides were modeled by the method using again the software PEPFOLD2.0 - RPBS (http://mobyle.rpbs.univ-paris-diderot.fr/cgi-bin/portal.py#forms::PEP-FOLD) and the filters from the program. The interactions (molecular docking) between the peptides and FabC4 were predicted by the HPEPDOCK software (http://huanglab.phys.hust.edu.cn/hpepdock/). The three-dimensional visualization files of the ANXA2 and Ck10 targets were in a protein database, more specifically the Protein Data Bank (https://www.rcsb.org/pdb).

. Molecular dockings were also performed, via PATCHDOCK (https://bioinfo3d.cs.tau.ac.il/PatchDock/), between proteins previously described as FabC4 targets (ARAÚJO, 2014) and the recombinant antibody. Finally, the prediction to assess whether the selected epitopes correspond to specific regions of these proteins, and all visualizations and analyzes were performed in the PyMol software.

## Claims

1. - Recombinant peptides that bind a tumor-specific antibody wherein it comprises sequences Seq. ID No. 1 to Seq. ID No. 4.

2. - Use of the selected peptides from Seq. ID No. 1 to Seq. ID No. 4 or any combination thereof, wherein they are used in the diagnosis of triple-negative breast cancer.

3. - Use of the selected peptides from Seq. ID No. 1 to Seq. ID No. 4 or any combination thereof, wherein they are used in the detection of breast cancer binding molecules through immunological assays, like enzyme immunoassays, such as ELISA, Western Blot, immunohistochemistry, immunofluorescence, and others, immunoagglutination tests, immunophenotyping , electrochemical sensors, flow cytometry, or any other form of detection related directly or indirectly to blood, urine, or saliva samples.

4. - Use of the selected peptides from Seq. ID No. 1 to Seq. ID No. 4 or any combination thereof, wherein they are used with radioisotope labeling, conjugated to fluorophores in general, Alexa-fluor variants with their different wavelengths, GFP (green fluorescent protein), PEG (polyethylene glycol), biotin, quantum-dots, or used together with labeled secondary antibodies in general for detection, and also used in conjunction with chemical groups and chemotherapeutic molecules in breast cancer treatment.

5. - Use of the selected peptides from Seq. ID No. 1 to Seq. ID No. 4 or any combination thereof, wherein they are used in conjunction with nanomaterials and reagents for the detection, characterization and staging of triple-negative breast cancer.

6. - Use of the selected peptides from Seq. ID No. 1 to Seq. ID No. 4 or any combination thereof, wherein they are labeled or conjugated to fluorescent, colorimetric, radioisotopes, luminescent, nanocrystals, magnetic nanoparticles, and other agents for *in vitro* or *in vivo* imaging, detection by fluorescence, luminescence, or colorimetry in static or portable equipment, and microscope viewing.

7. - Use of the selected peptides from Seq. ID No. 1 to Seq. ID No. 4 or any combination thereof, wherein they are biomarkers for triple-negative breast cancer, as a diagnostic method conjugated or not to nanomaterials and reagents for detection.

8. - Immunogenic Composition, wherein it comprises one or a combination of peptides selected from Seq ID No. 1 to Seq. ID No. 4 and a physiologically and pharmaceutically acceptable adjuvant.
